Europäisches Patentamt

European Patent Office

Office européen des brevets

(19)

(11) Publication number: **0 295 538**
**A2**

# EUROPEAN PATENT APPLICATION

(21) Application number: 88109056.7

(22) Date of filing: 07.06.88

(51) Int. Cl.⁴: **A61K 31/71 , A61K 31/70 , A61K 31/045 , A61K 31/435**

(30) Priority: 08.06.87 US 58962

(43) Date of publication of application:
21.12.88 Bulletin 88/51

(84) Designated Contracting States:
AT BE CH DE ES FR GB GR IT LI LU NL SE

(71) Applicant: **MERRELL DOW PHARMACEUTICALS INC.**
**2110 East Galbraith Road**
**Cincinnati Ohio 45215-6300(US)**

(72) Inventor: **Sunkara, Sai P.**
**9629 Linfield Drive**
**Cincinnati Ohio 45242(US)**
Inventor: **Bowlin, Terry L.**
**8466 Pond Ridge Drive**
**Maineville Ohio 45039(US)**
Inventor: **Liu, Paul S.**
**7370 Drake Road**
**Cincinnati Ohio 45243(US)**

(74) Representative: **Vossius & Partner**
**Siebertstrasse 4 P.O. Box 86 07 67**
**D-8000 München 86(DE)**

(54) Inhibitors of glycoprotein processing having anti retroviral activity.

(57) Castanospermine, swainsonine, deoxynojirimycin, deoxymannojirimycin, bromoconduritol, and tunicamycin, known inhibitors of glycoprotein processing, are disclosed to be effective in treating retroviral infections including HIV infections and are thus useful in the treatment of AIDS and ARC.

EP 0 295 538 A2

## INHIBITORS OF GLYCOPROTEIN PROCESSING HAVING ANTI-RETROVIRAL ACTIVITY

This invention relates to the use of certain inhibitors of glycoprotein processing in the treatment of retroviral infections including HIV infections.

A great deal of research is currently underway to develop treatments and cures for viral infections in humans and in animals. Notably the incidence of AIDS and ARC in humans is increasing at an alarming rate. The five year survival rate for those with AIDS is dispiriting and AIDS patients, whose immune systems have been seriously impaired by the infection, suffer from numerous opportunistic infections including Kaposi's sarcoma and *Pneumocystis carninii* pneumonia. No cure is known and current treatments are largely without adequate proof of efficacy and have numerous untoward side effects. Fear of the disease has resulted in social ostracism of and descrimination against those having or suspected of having the disease.

Retroviruses are a class of ribonucleic acid {RNA) viruses that replicate by using reverse transcriptase to form a strand of complementary DNA (cDNA) from which a double stranded, proviral DNA is produced. This proviral DNA is then randomly incorporated into the chromasomal DNA of the host cell making possible viral replication by later translation of the integrated DNA containing the viral genome.

Many of the known retroviruses are oncogenic or tumor causing. Indeed the first two human retroviruses discovered, denoted human T-cell leukemia virus I and II or HTLV-I and II, were found to cause rare leukemias in humans after infection of T-lymphocytes. The third such human virus to be discovered, HTLV-III, now referred to as HIV, was found to cause cell death after infection of T-lymphocytes and has been identified as the causative agent of acquired immune deficiency syndrome (AIDS) and AIDS related complex (ARC).

Retroviruses have, in addition to the usual viral capsid, an outer membrane of lipid and glycoprotein, similar to the membrane of ordinary cells. Indeed the lipid of the retroviral membrane is probably derived directly from the membrane of a previously infected host cell, however, the glycoprotein of the viral membrane is unique to the virus itself and is coded for by the viral genome. Infection of a host cell by a retrovirus initially relies on the interaction of various receptors on the host cell surface with the glycoprotein membrane envelope of the virus. Subsequently the virus and cell membranes fuse and the virion contents are released into the host cell cytoplasm. The glycoprotein envelope of the retroviruses is thought to play an important role in both the initial interaction of the virion and the host cell and in the later fusion of the viral and host cell membranes.

Interference with protein glycosylation could prevent the initial virus-host cell interaction or subsequent fusion or could prevent viral duplication by preventing the formation of the glycoprotein required for construction of the viral membrane. It has been recently reported that the glycosylation inhibitors 2-deoxy-D-glucose and 8-hydroxy-norvaline inhibit expression of HIV glycoproteins and block the formation of syncytia; see H.A. Blough et al., Biochemical and Biophysical Research Communications, 141(1), 33-38 (1986). Viral multiplication of HIV-infected cells treated with these agents is stopped, presumably because of the unavailability of glycoprotein required for viral membrane formation. In another report, the glycosylation inhibitor 2-deoxy-2-fluoro-D-mannose was found to exhibit antiviral activity against influenza infected cells by preventing the glycosylation of viral membrane protein; see W. McDowell et al.,Biochemistry, 24-(27), 8145-52 (1985). This report also studied the antiviral activity of 2-deoxyglucose and 2-deoxy-2-fluoroglucose and found that each inhibit viral protein glycosylation by a different mechanism. Many other known glycosylation inhibitors are known to have no antiviral activity. Thus the antiviral activity against membraned viruses, in general, and the anti-retroviral activity, specifically, of glycosylation inhibitors is quite unpredictable.

The applicants have now discovered that the known inhibitors of glycoprotein processing, castanospermine, swainsonine, deoxynojirimycin, deoxymannojirimycin, bromoconduritol, and tunicamycin are useful in the treatment of various retroviral infections including in the treatment of AIDS and ARC resulting from infection by HIV or other retroviruses.

The inhibitors of glycoprotein processing castanospermine, swainsonine, deoxynojirimycin, deoxymannojirimycin, bromoconduritol, and tunicamycin are anti-retroviral agents and can be used to treat the infections of retroviruses including HIV.

Castanospermine, 1,6,7,8-tetrahydroxyoctahydro-indolizine, (R. Saul, et al., Proc. Natl. Acad. Sci. USA, 1985, 82, 93-97; R. Saul, et al., Arch. Biochem. Biophys., 1983, 221(2), 593-597); swainsonine, octahydro-1,2,8-indolizinetriol, (A. D. Elbein, et al., Biochemistry, 1981, 78(12), 7393-97); deoxynojirimycin, 5-amino-5-deoxyglucose, (B. Saunier, et al., J. Biol. Chem., 1982, 257(23), 14155-161); deoxymannojirimycin (A. D. Elbein, et al., Arch. Biochem. Biophys., 1984, 235(2), 579-88); bromoconduritol, 6-bromo-3,4,5-trihydroxycyclohex-1-ene, (R. Datema, *et al.*, Proc. Natl. Acad. Sci. USA, 1982, 79, 6787-91); and tunicamycin, a mixture of at least 10 homologous antibiotics derived from the cultivation of *Streptomyces chartreusis*, (W. C. Mahoney and D. Duksin, J. Biol. Chem., 1979, 254(14), 6572-76) are known to be inhibitors of glycoprotein processing.

For each of the named inhibitors of glycoprotein processing, applicants mean not only the free base form but, where applicable, the pharmaceutically acceptable acid addition salts thereof. The expression "pharmaceutically acceptable acid addition salts" is intended to apply to any non-toxic organic or inorganic acid addition salts of the base compounds. Illustrative inorganic acids which form suitable salts include hydrochloric, hydrobromic, sulfuric, and phosphoric acids and acid metal salts such as sodium monohydrogen orthophosphate and potassium hydrogen sulfate. Illustrative organic acids which form suitable salts include the mono, di, and tricarboxylic acids. Illustrative of such acids are,for example, acetic, glycolic, lactic, pyruvic, malonic, succinic, glutaric, fumaric, malic, tartaric, citric, ascorbic, maleic, hydroxymaleic, benzoic, hydroxybenzoic, phenylacetic, cinnamic, salicylic, and 2-phenoxybenzoic acids. Other organic acids which form suitable salts are the sulfonic acids such as methane sulfonic acid and 2-hydroxyethane sulfonic acid. These salts and the base compounds can exist in either a hydrated or a substantially anhydrous form. The acid salts are prepared by standard techniques such as by dissolving the free base in aqueous or aqueous-alcohol solution or other suitable solvent containing the appropriate acid and isolating by evaporating the solution, or by reacting the free base in an organic solvent in which case the salt separates directly or can be obtained by concentration of the solution. In general the acid addition salts of the compounds of this invention are crystalline materials which are soluble in water and various hydrophilic organic solvents and which in comparison to their free base forms, demonstrate higher melting points and an increased solubility.

The ability of the glycosylation inhibitors of this invention to act as anti-retroviral agents can be demonstrated by their ability to inhibit the growth and replication of murine leukemia virus, an oncogenic retrovirus, as determined by an *in vitro* XC plaque assay. This assay was performed according to the method of Rowe *et al.* (Virology, 1970, 42, 1136-39) as previously described by L. Hsu, *et al.* (J. Virological Methods, 1980, 1, 167-77) and T. L. Bowlin and M. R. Proffitt (J. Interferon Res., 1983, 3(1), 19-31). Mouse SC-1 cells (fibroblast) ($10^5$) were seeded into each well of 6-well cluster plates (Costar #3506) in 4 ml Minimum Essential Medium (MEM) with 10% Fetal Calf Serum (FCS). Following an 18 hour incubation period (37°C), Moloney murine leukemia virus (MoLV) was applied at a predetermined titer to give optimal (i.e. countable) numbers of virus plaques. Compounds were added 2 hours prior to addition of the virus. Three days later the culture medium was removed, the SC-1 cell monolayers were exposed to UV irradiation (1800 ergs), and rat XC cells ($10^6$) were seeded into each well in 4 ml MEM. Following an additional 3 day incubation (37°C), these cells were fixed with ethyl alcohol (95%) and stained with 0.3% crystal violet. Plaques were then counted under low magnification. The antiviral activities of deoxynojirimycin and castanospermine, as quantitated in this test, are tabulated in Table 1.

| COMPOUND | $IC_{50}$ (ug/ml) |
|---|---|
| Deoxynojirimycin | 1.0 |
| Castanospermine | 1.0 |

The compounds of this invention can be used to treat a number of diseases and conditions known to be caused by retroviruses including those diseases and conditions caused by murine leukemia virus, feline leukemia virus, avian sarcoma virus, human immunodeficiency virus (HIV), HTLV-I, and HTLV-II. Those experienced in this field are readily aware of the circumstances requiring anti-retroviral therapy. Applicants consider the use of the compounds of this invention, and especially the use of castanospermine, to treat HIV infections in humans to be of most importance. The term "patient" used herein is taken to mean mammals such as primates, including humans, sheep, horses, cattle, pigs, dogs, cats, rats and mice.

The amount of the inhibitor of glycoprotein processing to be administered can vary widely according to the particular dosage unit employed, the period of treatment, the age and sex of the patient treated, the nature and extent of the disorder treated, and the particular inhibitor of glycoprotein processing selected. Moreover the inhibitor of glycoprotein processing can be used in conjunction with other agents known to be useful in the treatment of retroviral diseases and agents known to be useful to treat the symptoms of and

3

complications associated with diseases and conditions caused by retroviruses. The anti-retrovirally effective amount of an inhibitor of glycoprotein processing to be administered will generally range from about 15 mg/kg to 500 mg/kg. A unit dosage may contain from 25 to 500 mg of the inhibitor of glycoprotein processing, and can be taken one or more times per day. The inhibitor of glycoprotein processing can be administered with a pharmaceutical carrier using conventional dosage unit forms either orally or parenterally.

The preferred route of administration is oral administration. For oral administration the inhibitors of glycoprotein processing can be formulated into solid or liquid preparations such as capsules, pills, tablets, troches, lozenges, melts, powders, solutions, suspensions, or emulsions. The solid unit dosage forms can be a capsule which can be of the ordinary hard- or soft-shelled gelatin type containing, for example, surfactants, lubricants, and inert fillers such as lactose, sucrose, calcium phosphate, and cornstarch. In another embodiment the compounds of this invention can be tableted with conventional tablet bases such as lactose, sucrose, and cornstarch in combination with binders such as acacia, cornstarch, or gelatin, disintegrating agents intended to assist the break-up and dissolution of the tablet following administration such as potato starch, alginic acid, corn starch, and guar gum, lubricants intended to improve the flow of tablet granulations and to prevent the adhesion of tablet material to the surfaces of the tablet dies and punches, for example, talc, stearic acid, or magnesium, calcium, or zinc stearate, dyes, coloring agents, and flavoring agents intended to enhance the aesthetic qualities of the tablets and make them more acceptable to the patient. Suitable excipients for use in oral liquid dosage forms include diluents such as water and alcohols, for example, ethanol, benzyl alcohol, and the polyethylene alcohols, either with or without the addition of a pharmaceutically acceptably surfactant, suspending agent, or emulsifying agent.

The compounds of this invention may also be administered parenterally, that is, subcutaneously, intravenously, intramuscularly, or interperitoneally, as injectable dosages of the compound, in a physiologically acceptable diluent with a pharmaceutical carrier which can be a sterile liquid or mixture of liquids such as water, saline, aqueous dextrose and related sugar solutions, an alcohol such as ethanol, isopropanol, or hexadecyl alcohol, glycols such as propylene glycol or polyethylene glycol, glycerol ketals such as 2,2-dimethyl-1,3-dioxolane-4-methanol, ethers such as poly(ethyleneglycol) 400, an oil, a fatty acid, a fatty acid ester or glyceride, or an acetylated fatty acid glyceride with or without the addition of a pharmaceutically acceptable surfactant such as a soap or a detergent, suspending agent such as pectin, carbomers, methylcellulose, hydroxypropylmethylcellulose, or carboxymethylcellulose, or emulsifying agent and other pharmaceutically adjuvants. Illustrative of oils which can be used in the parenteral formulations of this invention are those of petroleum, animal, vegetable, or synthetic origin, for example, peanut oil, soybean oil, sesame oil, cottonseed oil, corn oil, olive oil, petrolatum, and mineral oil. Suitable fatty acids include oleic acid, stearic acid, and isostearic acid. Suitable fatty acid esters are, for example, ethyl oleate and isopropyl myristate. Suitable soaps include fatty alkali metal, ammonium, and triethanolamine salts and suitable detergents include cationic detergents, for example, dimethyl dialkyl ammonium halides, alkyl pyridinium halides, and alkylamines acetates; anionic detergents, for example, alkyl, aryl, and olefin sulfonates, alkyl, olefin, ether, and monoglyceride sulfates, and sulfosuccinates; nonionic detergents, for example, fatty amine oxides, fatty acid alkanolamides, and polyoxyethylenepolypropylene copolymers; and amphoteric detergents, for example, alkyl-beta-aminopropionates, and 2-alkylimidazoline quarternary ammonium salts, as well as mixtures. The parenteral compositions of this invention will typically contain from about 0.5 to about 25% by weight of the inhibitor of glycoprotein processing in solution. Preservatives and buffers may also be used advantageously. In order to minimize or eliminate irritation at the site of injection, such compositions may contain a non-ionic surfactant having a hydrophile-lipophile balance (HLB) of from about 12 to about 17. The quantity of surfactant in such formulations ranges from about 5 to about 15% by weight. The surfactant can be a single component having the above HLB or can be a mixture of two or more components having the desired HLB. Illustrative of surfactants used in parenteral formulations are the class of polyethylene sorbitan fatty acid esters, for example, sorbitan monooleate and the high molecular weight adducts of ethylene oxide with a hydrophobic base, formed by the condensation of propylene oxide with propylene glycol.

EXAMPLE 1

Tablets are prepared each having the composition:

Castanospermine  250 mg

starch  40 mg

talc  10 mg

magnesium stearate  10 mg

EXAMPLE 2

Capsules are prepared each having the composition:

Deoxynojirimycin  400 mg

talc  40 mg

sodium carboxymethylcellulose  40 mg

starch  120 mg

EXAMPLE 3

Injectable dosages forms are prepared each having the composition:

Swainsonine  0.500 g

polyoxyethylene sorbitan monooleate  2.000 g

sodium chloride  0.128 g

water for injection qs ad  20.000 ml

**Claims**

1. An inhibitor of glycoprotein processing selected from castanospermine, swainsonine, deoxynojirimycin, deoxymannojirimycin, bromoconduritol, and tinucamycin for use as a medicament in a method of treating a retroviral infection.

2. An inhibitor of glycoprotein processing selected from castanospermine, swainsonine, deoxynojirimycin, deoxymannojirimycin, bromoconduritol, and tunicamycin for use as a medicament in a method of treating HIV infections.

3. The use of an inhibitor of glycoprotein processing selected from castanospermine, swainsonine, deoxynojirimycin, deoxymannojirimycin, bromoconduritol, and tunicamycin for the preparation of a medicament for the treatment of a retroviral infection.

4. The use of an inhibitor of glycoprotein processing selected from castanospermine, swainsonine, deoxynojirimycin, deoxymannojirimycin, bromoconduritol, and tunicamycin for the preparation of a medicament for the treatment of HIV infections.

5. Embodiment according to one of claims 1 to 4 wherein the inhibitor of glycoprotein processing is castanospermine.

6. Embodiment according to one of claims 1 to 4 wherein the inhibitor of glycoprotein processing is swainsonine.

7. Embodiment according to one of claims 1 to 4 wherein the inhibitor of glycoprotein processing is deoxynojirimycin.

8. Embodiment according to one of claims 1 to 4 wherein the inhibitor of glycoprotein processing is deoxymannojirimycin.

9. Embodiment according to one of claims 1 to 4 wherein the inhibitor of glycoprotein processing is bromoconduritol.

10. Embodiment according to one of claims 1 to 4 wherein the inhibitor of glycoprotein processing is tunicamycin.